(19) Europäisches Patentamt
European Patent Office
Office européen des brevets

(11) **EP 4 336 180 A1**

(12) **EUROPEAN PATENT APPLICATION**

(43) Date of publication:
**13.03.2024 Bulletin 2024/11**

(21) Application number: **22194277.4**

(22) Date of filing: **07.09.2022**

(51) International Patent Classification (IPC):
**G01N 33/00** *(2006.01)*    **A61B 5/08** *(2006.01)*
**A61B 5/097** *(2006.01)*    *G01N 33/497* *(2006.01)*

(52) Cooperative Patent Classification (CPC):
**G01N 33/0009; A61B 5/08; A61B 5/097;**
G01N 2033/4975

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Microfeeder A/S**
**8541 Skødstrup (DK)**

(72) Inventor: **FOGED, Henning Lyngsø**
**8541 Skødstrup (DK)**

(74) Representative: **Patrade A/S**
**Ceresbyen 75**
**8000 Aarhus C (DK)**

(54) **APPARATUS FOR MEASURING AERIAL GAS CONCENTRATIONS FROM SPECIFIC SOURCES, METHOD THEREFORE AND USE HEREOF**

(57)    The present invention relates to an aggregate for monitoring of aerial gas concentrations from specific sources, including production units. The present invention furthermore relates to a method of monitoring concentration of gases in air and the use of the aggregate for gas monitoring.

EP 4 336 180 A1

**Description**

**Field of the Invention**

The present invention relates to an aggregate for monitoring of aerial gas concentrations from specific sources, including production units. The present invention furthermore relates to a method of monitoring concentration of gases in air and the use of the aggregate for gas monitoring.

**Background of the Invention**

Known inventions for measuring aerial gas concentrations are integrated parts of wider inventions, or meant for monitoring ambient air quality, not emissions from specific sources, such as emissions from production units. For instance, the invention: "Method and system for monitoring and reducing ruminant methane production" as described in the patent document US7966971, which is a concentrate feed automate designed for dosing feed that reduces the measured enteric methane production of the animals, based on their sensed, individual exhale of methane or $CO_2$, uses a gas sensor as a minor component of a larger system, having a broader purpose.

**Object of the Invention**

One objective is to achieve a aggregate or apparatus for monitoring gas concentrations of air exhaled by production units, such as gas concentrations in ventilation air or animal breath.
A further objective is to achieve an aggregate or apparatus serving as a decision support tool, for instance in connection with management of commercial operations, including for example the compliance with legal limits for gas concentrations in production units, legal limits for gas emissions from production units, or for controlling gas emissions in or stemming from such production units, which may have relevance for the business management.

**Description of the Invention**

One objective is achieved by an apparatus for monitoring concentration of gases in air. The apparatus comprises an air collection unit configured to collect and sample air in a controlled environment.
The apparatus furthermore comprises a sensor chamber comprising one or more gas sensors.
The apparatus furthermore comprises a passive air sampler comprising a tube and an air pump with a suction side and a pressure side. The passive air sampler is arranged in communication with the air collection unit at the suction side and with the sensor chamber at the pressure side, such that the passive air sampler is configured for dragging air from the air collection unit to the sensor chamber for collecting air samples from the air collection unit.
The one or more gas sensors may be of various types, shapes and sizes.
The one or more gas sensors are arranged in adjustable fixtures, and the sensor chamber is configured for replacement of the one or more gas sensors.
The collected air samples in the sampling chamber are brought into contact with the one or more replaceable gas sensors for real time gas monitoring.
The sensor chamber may by a substantially airtight sensor chamber, configured with an airtight opening, openings for tubes for inlet and outlet of air, and adjustable fixtures for one or more gas sensors.
The present invention is a separate, individual apparatus that makes it possible to employ gas sensors of various types, sizes and shapes. It is intended for environmental and business management purposes in cases where it is relevant to monitor the emission of certain gases from specific sources, such as production units, including post adoptatus in or at production units.
Gas sensors are electronic components available on the market per se, or, most often offered in a form where they are integrated with a small circuit board for controlling the power supply to the sensor and the reading out of sensor signals. Gas sensors offered on the market are thus not ready for being employed in practical use but supposed to be used as integrated components of various specific equipment, such as is the case described in patent document US7966971.
The present invention makes it possible in a simple, quick and easy way to employ gas sensors of various types, shapes and sizes, and attach them to specific production units for the purpose of monitoring emissions of specific sources, only requiring a minimum of adoption in the settings of the invention, such as settings for controlling the air flow of the sampling unit to adapt the gas concentration in the sampled air to the upper and lower range of the used gas sensors' sensing range.
The background for the invention is known technology for identifying animals that are equipped with devices for radio frequency identification, such as electronic ear tags, software for registration of individual animals visit such as described in WO2020020423 - "System for precision mineral supplementation of animals". Such known technology can be used in combination with the invention in case the production unit is an animal, and there is a wish to establish a real-time online monitoring using a data flow communication via the cloud.

The disclosed aggregate can via interpretation of signals from its gas sensors monitor the concentration of gases, such as ammonia ($NH_3$), methane ($CH_4$), carbon dioxide ($CO_2$), or ketones, in the air. The aggregate can be used for monitoring air of production units, such as ventilation air from buildings or individual animal breath.

Aggregate and apparatus is used interchangeably throughout this patent application.

A production unit is in this connection defined as an inventory used for production, such as a production facility, a building, a machine, or an animal.

One purpose of the aggregate may be to serve as a decision support tool, for instance in connection with management of commercial operations, including for example the compliance with legal demands to the air quality in production units, or legal limits to emissions from production units, in all cases where the production unit is a source of gas emissions that cause the concentration of such gases in the air deviate from the concentration of these gases in the ambient air.

The aggregate can for instance be used for monitoring the concentration of specific gases at a production unit, including polluting gases, such as the ammonia concentration of air in livestock stables or in the ventilation air from stables. The ammonia concentrations in the air of a production unit, or in the ventilation air of a production unit, reflects the emission of the gas from that unit, which can have both environmental and economic aspects, especially in cases where the operation of a production unit is based on an environmental approval.

The aggregate can also be used for passive and non-invasive monitoring of gases in animal breath, in which case the production unit is an individual animal. Certain gases are indicators for respiratory and metabolic diseases, such as phenol, benzothiazole, p-cresol and 5-octadecanal that are indicators for respiratory disease, or ketones, for example acetone, that are indictors for ketosis. Monitoring such gases can secure an early diagnosis and treatment of the animal at the sub-clinical stage, thus reducing the economic losses of diseases. Another example is the monitoring of individual cow breath for detection of the concentration of enteric methane in cow burps, an information that considering its high heritability of around 0.4 is instrumental in cattle breeding and for the practical culling management in the herds to reduce the greenhouse gas footprint of cattle production and its possible economic implications. Yet another example is the possibility to monitor sulphides (for example hydrogen sulphide; dimethyl sulphide) - that are indicators of excess protein intake/protein degradation.

The invention may provide for monitoring of all types of gases in or exhaled by production units, and the above mentioned are alone to be considered as examples.

The aggregate for monitoring the concentration of gases in air is versatile, meaning it can be used as an individual aggregate in case it is envisaged to monitor gases emitted by production units or in the ventilation air of production units.

This means that the aggregate may be located and fixed in relation to various types of production units according to the relevant place for monitoring the gas in question. Furthermore, the invention can be equipped with gas sensor type(s) with respect to sensing technology, price and preciseness according to the envisaged purpose for monitoring specific gases.

The design and components of the invention secures the possibility for a standardised sampling and sensing of gases in air of production units, including the possibility to control the air flow of the air around the sampling place, physically protect the air to be sampled from for example rain, and control the sampling rate, such as the volume of sampled air over time for a specified period.

The ability to control the air flow of the air collection unit of the aggregate may have the specific purpose to enable the conversion of the monitored gas concentration to a quantified gas emission for a given period.

For example, if a gas concentration is measured as 750 ppm (average of measurements), the gas having a density of 0.657 kg/m3 (methane) and the air flow in the air collection unit is 100 litres per minute, the quantified emission is easily converted to an annual volume-based production:

$$100 \text{ litres/minute} * 565,600 \text{ minutes/year} * 750/1,000,000 / 1,000 \text{ litres/m3} = 39.42 \text{ m3/year}$$

This can further be re-calculated to a weight based annual production:

$$39.42 \text{ m3/year} * 0.657 \text{ kg/m3} = 25.89894 \text{ kg/year}$$

This example illustrates the importance of the ability to control the air flow, without which the gas concentrations could not be converted into a weight-based gas production from the given specific source, such as a production unit, neither adjust the gas concentration of the sampled air to be within the upper and lower detection range for the used gas sensor. Furthermore, the ability to manage the air flow of the air collection unit also means that it is possible to drag air through the unit directly from the source for the gas emission. In case it is important to use the invention for measuring a gas emission in terms of a weight-based production, it is important the air collection unit is positioned so that all emitted gas is dragged through the unit as long as the measurement is taking place. In some cases, according to the relevance and possibilities, the production units will not have their emitted gas monitored constantly, but the results of the measurements for a given sampling time be prolonged to represent an entire time unit production. This is in specific the case when measurements are performed on animal breath, as the animals will not have all their breath sampled because they would move around in the stable or be outside during grazing.

The disclosed aggregate comprises amongst other a control box that holds the used gas sensor(s), situated in a sensor chamber, as well as other electronic and electrical components and hardware.

Gas sensors operates according to different sensing technologies, they can detect specific gases or gas types. The gas sensors have different sizes, but are typically rather small, typically having sizes measured in mm.

Typically, the gas sensors are mounted on circuit boards holding electronic components for the purpose of supplying the sensor with electricity and collecting measured data on memory cards and/or transmitting it to the cloud.

The circuit boards may be rectangular shaped, having a thickness of 1-2 mm and a width and length under 10 cm.

Sensors can be selected according to their price and preciseness according to its foreseen use, with commercial use sensor types typically having lower preciseness and price, whereas those intended for scientific purposes or where the monitoring has legal implications, would be selected to be more precise and would be higher priced. The invention is versatile in the way that it due to the design of the control box can be equipped with sensors of various sizes, shapes, and types.

The control box also holds an air pump to lead the monitored air from the air collection unit to the sensor chamber at a defined speed, where the gas sensor is located. The air collection unit offers possibility for managing the rate of the dilution of the monitored gas with ambient air in case the gas source is located at a specific point. Specifically, the control box makes it possible to manage the speed of the ventilator of the air collection unit to an envisaged speed that results in an envisaged air flow through the air collection unit, so that the air flow results in a gas concentration of the sampled air that complies with the detection range limits for the used gas sensor, and so that the air flow is sufficient for collection of the entire gas emission form the given source, meaning the air flow of the air collection unit must be higher than the emitted gas volume from the specific production unit source. The air flow of the air collection unit is determined via the adjustable speed of its ventilator, giving a known relation between the speed of the ventilator and the airflow in the air collection unit.

Components of the invention

The disclosed aggregate may hold one or more of the following components:

- A control box for holding various electrical and electronical components, including replaceable gas sensor(s) situated in a sensor chamber, designed so that gas sensors of various types, and therefore of different sizes and shapes can be used. Its design allows users' frequent replacement of the gas sensors to ensure the preciseness of the invention over time in cases where a calibration of a sensor would be impossible or less advantageous. The control box has a door for easy opening, and it has water and dust ingress protection (IP) rating that suits the production unit from which air is sampled, for instance IP68 in case of moist and dusty locations. The easy opening of the control box is secured by a door that is hinged in one side and having a snap-lock on the other side of the door, which can be manually opened without the use of tools.

- An air collection unit of a relevant geometrical shape ensure that the air is collected in a controlled environment, and for instance frequently exchanged for a reliable real time monitoring, and so that the disturbance of the sampling by rain, moist or dust is reduced. In cases where the gas to be monitored stems from a specific source, such as a building, a machine or an animal, which exhaust air that has a considerable higher concentration of the specific gas than the ambient air, it is important for the quality of the monitoring that all the gas from the source is collected. The air sampling /collecting unit is designed with a ventilator that allow to manage the air flow, and thus drag the air from the source into the air sampling / collection unit. Likewise, the ventilator of the air sampling / collection unit allows the control with the air flow, and thus the dilution of the gas from the source with the ambient air. The management of the dilution is important to ensure the samples air that is led to the sensor chamber has a gas concentration within the sensing limits of the used gas sensor. Gas sensors are typically having upper and lower limits for their ability to sense the gases in question in a precise way.

- A passive air sampler, comprising of an air pump that via a tube drags air from the air collection unit to the sensor chamber.

- A sensor chamber for holding the gas sensor. The sensor chamber is airtight, and it is designed with an air inlet and outlet, and with an airtight passage for wires for power and data transmission. The sensing chamber holds a fixture for circuit boards with the gas sensors, which is adjustable for various sizes of circuit boards, and which allow easy and manual replacement of the circuit boards with gas sensors in the control box without the use of tools. The invention can be designed in the way that the fixture is part of the opening of the of the sensor chamber, and it has a fixed slot in one side for the circuit board, and an adjustable fixture on the other side. The sensor chamber can be easily opened for replacement of the gas sensor, the opening is equipped with a rubber gasket or alike for keeping the sensor chamber airtight when closed.

- A replaceable gas sensor, typically integrated with a circuit board with electronic components for supplying the gas sensor with electricity and for capturing sensor data and either store this or communicate it to the cloud. The invention may be used with relatively cheap gas sensors for commercial use, whereas it can also operate with more expensive and precise sensors for scientific purposes or where this is otherwise relevant.

- Air pump for a controlled sampling from the air collection /sampling unit.

- Sensor chamber, where the sampled air is brough into contact with the sensor.

- Hardware in accordance with the relevant configuration, such as power converter, comprising also a turn switch for control of the speed of the ventilator of the air collection unit, a display to show the actual air flow through the air collection unit, and electronics for sensor data exchange.

In practice, the invention appears as two units, an air collection unit and a control box holding several components, connected by a tube for leading sampled air from the air collection unit to the sensor chamber inside the control box.

## Description of the Drawing

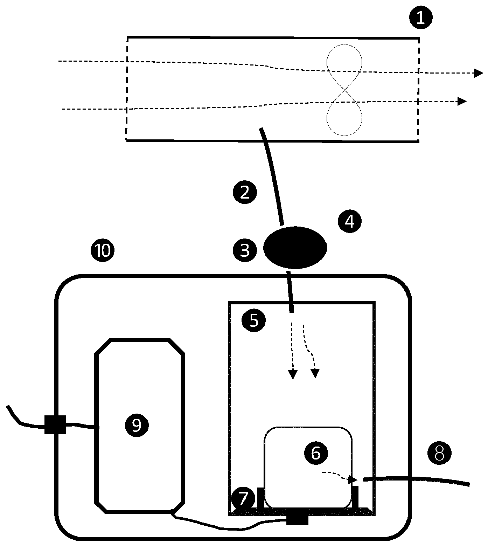

Fig. 1 illustrates an apparatus according to the invention.

## Detailed Description of the Invention

| No. | Item |
| --- | --- |
| 1 | air collection unit |
| 2 | tube |
| 3 | air pump |
| 4 | passive air sampler |
| 5 | sensor chamber |
| 6 | gas sensor |
| 7 | fixture for gas sensor |
| 9 | hardware |
| 10 | control box |
| 100 | Apparatus |

Figure 1 illustrates one exemplary embodiment of the aggregate 100 comprising a control box 10 for holding various electrical and electronical components, including replaceable gas sensor(s) 6. The gas sensors are arranged in a sensor chamber 5 designed so that gas sensors of various types, and therefore of different sizes and shapes can be used.

The design of the control box 10 and the sensor chamber 5 allows for frequent replacement of the gas sensors to ensure accuracy of the aggregate over time. Especially in cases where a calibration of a sensor would be impossible or less advantageous.

The control box may have a door for easy opening, and water and dust ingress protection (IP) rating that suits the production unit from which air is sampled, for instance IP68 in case of moist and dusty locations.

The illustrated embodiment furthermore comprises an air collection unit 1 with a geometrical shape ensuring that the air is collected in a controlled environment. The air collection unit may furthermore be designed to ensure frequently exchange of the collected air for a reliable real time monitoring, and to ensure that the disturbance of the sampling by rain, moist or dust is reduced.

The illustrated embodiment furthermore comprises a passive air sampler 4 comprising an air pump 3 and a tube 2. The passive air sampler 4 drags air from the air collection unit 1 to the sensor chamber 5.

The control box 10 may furthermore comprise various hardware 9 for specific embodiments of the aggregate for relevant configuration, such as power converter, switches for control of the speed of the ventilator of the air collection unit 1, a display to show the actual air flow through the air collection unit 1 and/or electronics for sensor data exchange.

In one embodiment, the invention may appear as two units, an air collection unit 1 and a control box 10 holding several components connected by a tube 2 for leading sampled air from the air collection unit 1 to the sensor chamber 5 inside the control box 10.

**Claims**

1. Apparatus (100) for monitoring concentration of gases in air, said apparatus comprising:

   • an air collection unit (1) configured to sample and collect air in a controlled environment;
   • a sensor chamber (5) comprising one or more gas sensors (6), and
   • a passive air sampler (4) comprising a tube (2) and an air pump (3) with a suction side and a pressure side, said passive air sampler (4) being arranged in communication with the air collection unit (1) at the suction side and the sensor chamber (5) at the pressure side, such that the air sampler is configured for dragging air from the collection unit (1) to the sensor chamber (5) for collecting air samples from the air collection unit (1), wherein the one or more gas sensors (6) are arranged in adjustable fixture(s) (7), and the sensor chamber is configured for replacement of the one or more gas sensors (6), and wherein the collected air samples in the sampling chamber (5) are brought into contact with the one or more replaceable gas sensors (6) for real time gas monitoring.

2. The apparatus according to claim 1, wherein the air collection unit (1) is configured for supporting a managed air flow with respect to velocity and avoidance of rain, moisture and dust.

3. The apparatus according to any one of claims 1 or 2, wherein the one or more replaceable gas sensors (6), possibly integrated with a circuit board having electronic components for supplying the gas sensors with electricity, for collecting sensor data and for storing or communicating the collected sensor data, are located at adjustable fixtures (7) that allow the use and easy replacement of gas sensors of various sizes, shapes and types.

4. The apparatus according to any one or more of the preceding claims, wherein the replaceable sensors (6) are adapted for sensing relevant types of gases in or exhaled by production units, such as ammonia ($NH_3$), methane ($CH_4$), carbon dioxide ($CO_2$) or other gases related to air quality and greenhouse gas emissions.

5. The apparatus according to any one or more of the preceding claims, wherein the air collection unit (1) comprises a ventilator controlling air flow through the air collection unit (1).

6. The apparatus according to claim 5, wherein the apparatus comprises a control box (10) configured to control the speed of the ventilator to ensure the gas concentration of the sampled air is within the detection range limits of the one or more gas sensors (6) and ensure the air flow is sufficient for collection of qualitative monitoring of the emitted gas from a production unit source.

7. The apparatus according to claim 6, wherein the sensor chamber (5) is positioned within the control box (10).

8. The apparatus according to any one or more of the preceding claims, wherein the sensor chamber comprises an outlet for sampled air.

9. Method of monitoring concentration of gases in air comprising acts of

   - continuously collecting air from a controlled environment;
   - collecting air sample from the collected air;

- transferring the collected air sample to a sampling chamber, and
- bringing the collected air samples into contact with one or more replaceable gas sensors (4) for analysing the air samples for content of one or more gasses.

10. The method according to claim 9 using the apparatus (100) according to any one or more of claims 1-8.

11. Use of the apparatus (100) according to any one or more of claims 1-8 for passive and non-invasive monitoring of gases in animal breath.

12. Use according to claim 11 wherein the monitoring is for phenol, benzothiazole, p-cresol and 5-octadecanal, sulphides (e.g. hydrogen sulphide; dimethyl sulphide), amines (e.g. putrescine, methylamines), ketones (e.g. acetone) or other gases that are indicators for animal health.

13. Use according to any one of claims 11 or 12 for detection of respiratory and/or metabolic diseases in animals.

# EP 4 336 180 A1

Europäisches Patentamt
European Patent Office
Office européen des brevets

## EUROPEAN SEARCH REPORT

**Application Number**

EP 22 19 4277

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2022/205965 A1 (HUR SANG-HOON [KR] ET AL) 30 June 2022 (2022-06-30) * paragraphs [0001] - [0085]; figures 1-15 * | 1-4,8-10 | INV. G01N33/00 A61B5/08 A61B5/097 |
| X | US 2011/208081 A1 (SMITH TREVOR [GB] ET AL) 25 August 2011 (2011-08-25) * paragraphs [0001] - [0085]; figures 1-4 * | 1-13 | ADD. G01N33/497 |
| X | CN 114 994 299 A (INTELLIGENT EQUIPMENT TECH CENTER BEIJING ACADEMY OF AGRICULTURE AND F) 2 September 2022 (2022-09-02) * paragraphs [0001] - [0174]; figures 1-15 * | 1-13 | |
| X | PLACE SARA E. ET AL: "Construction and Operation of a Ventilated Hood System for Measuring Greenhouse Gas and Volatile Organic Compound Emissions from Cattle", ANIMALS, vol. 1, no. 4, 1 December 2011 (2011-12-01), pages 433-446, XP055838059, ISSN: 2076-2615, DOI: 10.3390/ani1040433 * pages 1-14; figures 1-11; table 1 * | 1,2,4-13 | |

**TECHNICAL FIELDS SEARCHED (IPC)**

G01N
A61B

The present search report has been drawn up for all claims

| Place of search | Date of completion of the search | Examiner |
|---|---|---|
| The Hague | 3 February 2023 | Gassmann, Martin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

## ANNEX TO THE EUROPEAN SEARCH REPORT
## ON EUROPEAN PATENT APPLICATION NO.

EP 22 19 4277

This annex lists the patent family members relating to the patent documents cited in the above-mentioned European search report.
The members are as contained in the European Patent Office EDP file on
The European Patent Office is in no way liable for these particulars which are merely given for the purpose of information.

03-02-2023

| Patent document cited in search report | | Publication date | Patent family member(s) | | Publication date |
|---|---|---|---|---|---|
| US 2022205965 | A1 | 30-06-2022 | CN 109521076 | A | 26-03-2019 |
| | | | KR 101960522 | B1 | 20-03-2019 |
| | | | US 2019086379 | A1 | 21-03-2019 |
| | | | US 2022205965 | A1 | 30-06-2022 |
| US 2011208081 | A1 | 25-08-2011 | EP 2185928 | A1 | 19-05-2010 |
| | | | US 2011208081 | A1 | 25-08-2011 |
| | | | WO 2009030957 | A1 | 12-03-2009 |
| CN 114994299 | A | 02-09-2022 | NONE | | |

EPO FORM P0459

For more details about this annex : see Official Journal of the European Patent Office, No. 12/82

**REFERENCES CITED IN THE DESCRIPTION**

**Patent documents cited in the description**

- US 7966971 B
- WO 2020020423 A